# EUROPEAN PATENT APPLICATION

(11) **EP 1 367 049 A1**
(43) Date of publication of application: **03.12.2003**
(21) Application number: 02711425.5
(22) Date of filing: 08.02.2002
(51) Int. Cl.: C07C 327/44, C07D 277/30

(54) **CYANOTHIOACETAMIDE DERIVATIVE AND PROCESS FOR PRODUCING THE SAME**

(30) Priority: 13.02.2001 JP 2001034839; 05.04.2001 JP 2001106924
(71) Applicant: NIPPON SODA CO., LTD., Chiyoda-ku, Tokyo 100-8165 (JP)
(72) Inventor: YAGIHARA, Tomio, c/o Nippon Soda Co., Ltd, Odawara-shi, Kanagawa 250-0280 (JP); OOKUCHI, Tetsushi, c/o Nippon Soda Co., Ltd, Nakakubiki-gun, Niigata 949-2392 (JP)
(74) Representative: Schmitz, Jean-Marie
(86) International application number: JP0201099
(87) International publication number: WO02064554

(57) **Abstract**

A thioacetamide compound represented by the formula (1) (1) which is useful as an intermediate for an acrylonitrile derivative serving as an active ingredient for medicines and agricultural chemicals, especially insecticides/acaricides; a process for producing the compound; and a process for producing a 3-oxo-2-(2-thiazolyl)propanenitrile compound, characterized by reacting the compound represented by the formula (1) with phenacyl halide.

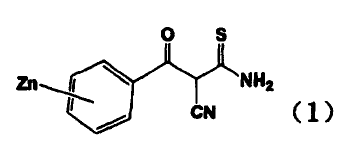

## Description

### Technological Fields:

The present invention relates to thioacetamide compounds useful as intermediates for producing acrylonitrile derivatives that are active ingredients for medicine and agricultural chemicals, particularly for insecticides and acaricides, to processes for producing the compounds, and to processes for the preparation of 3-oxo-2-(2-thiazolyl)-propanenitrile compounds from the said compounds.

### Background Art:

As for thioacetamide compounds analogous to the compounds of the present invention, N-substituted-á-aroyl-á-cyano-thioacetamides are used as starting materials for synthesizing 1,3-oxazoles [Z. Chem., 19,251 (1979)]. However, N-unsubstituted compounds, that is, benzoyl-substituted cyanothioacetamides, of the present invention are not described.

A known method for thioamidating nitriles with thioacetamide is that a simple nitrile compound is reacted with thioacetamide in dimethylformamide in the presence of hydrochloric acid to give the corresponding thioamide [J. Am. Chem. Soc., 82, 2656 (1960)]. No examples of using compounds with carbonyl groups that are easily sulfurized, such as those of the present invention, are described.

As for a process for producing 3-oxo-2-(2-thiazolyl)propanenitriles of the present invention, a method represented by the following reaction scheme is disclosed in EP 189960, WO 00/17174 and others.

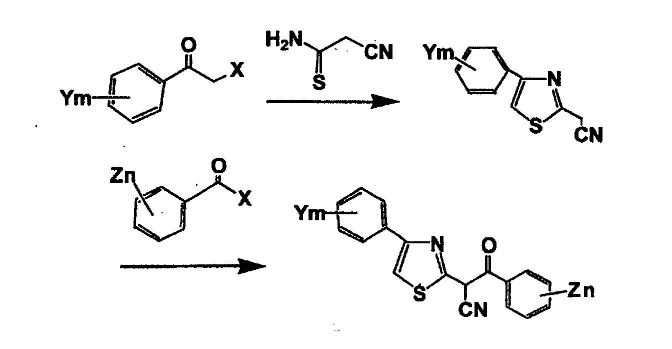

It is a process that cyanothioacetamide is reacted with a phenacyl halide to derive to a 2-cyanomethylthiazole compound, which is then benzoylated with a benzoyl halide to give the target compound.

This process produces the target compound in relatively high yield, but is disadvantageous in industrial mass production because cyanothioacetamide used as a starting material is expensive.

### Disclosure of the Invention

It is an object of the present invention to provide compounds that are expected to have biological activities useful in the fields of agricultural chemicals and medicine, and industrially advantageous processes for producing the same.

The inventors carried out reactions of benzoylmalononitrile with thioacetamide according to the method described in the aforementioned paper, with the products in low yields (Comparative Examples 1 and 2). Studies of the reactions in more detail have revealed that a target compound can be produced in high yield if reaction solvents and types of catalysts are properly selected to use, without using dimethylformamide (DMF) and hydrochloric acid, as is the case of the method described above.

The present invention firstly relates to a novel compound represented by Formula (1) 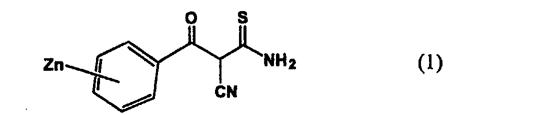 (wherein, Z is nitro, cyano, halogen, alkyl having 1 to 6 carbons, haloalkyl having 1 to 6 carbons, alkoxy having 1 to 6 carbons, optionally substituted phenyl or optionally substituted phenoxy; and n is 0 or an integer of 1 to 5).

Secondly, the present invention relates to a process for producing the compound of Formula (1) characterized in that a benzoylmalononitrile represented by Formula (2) 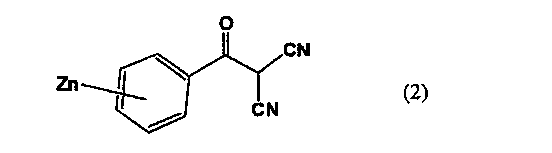 (wherein, Z and n are as defined above) is reacted with thioacetamide represented by Formula CH₃CSNH₂ in the presence of an acid.

Thirdly, it relates to a process for the preparation of a compound represented by Formula (4) 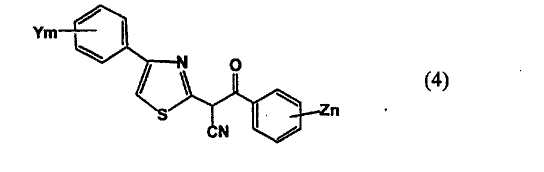 (wherein, Y is halogen, alkyl having 1 to 6 carbons, alkoxy having 1 to 6 carbons or optionally substituted phenyl; m is 0 or an integer of 1 to 5; and Z and n are as defined above) characterized in that a compound of Formula (1) is reacted with a phenacyl halide represented by Formula (3) 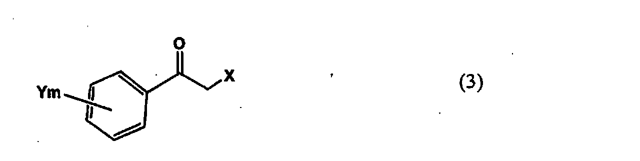 (wherein, X is halogen, and Y and m are as defined above).

### Forms to Implement the Invention:

The present invention is described in detail.

In the compounds of Formula (1) of the present invention, Z is cyano; nitro; halogen such as fluorine, chlorine or bromine; alkyl having 1 to 6 carbons such as methyl, ethyl, propyl, isopropyl, butyl, sec-butyl, isobutyl, t-butyl, pentyl or its isomer, or hexyl or its isomer; haloalkyl having 1 to 6 carbons such as chloromethyl, fluoromethyl, bromomethyl, dichloromethyl, difluoromethyl, dibromomethyl, trichloromethyl, trifluoromethyl, tribromomethyl, 2,2,2-trichloroethyl, 2,2,2-trifluoroethyl or pentafluoroethyl; alkoxy having 1 to 6 carbons such as methoxy, ethoxy, propoxy, isopropoxy, butoxy, sec-butoxy, isobutoxy or t-butoxy; phenyl optionally substituted with nitro, halogen such as fluorine and chlorine, alkyl having 1 to 6 carbons such as methyl and ethyl, or alkoxy having 1 to 6 carbons such as methoxy, ethoxy and isopropoxy; or phenoxy optionally substituted with nitro, halogen such as fluorine and chlorine, alkyl having 1 to 6 carbons such as methyl and ethyl, or alkoxy having 1 to 6 carbons such as methoxy, ethoxy and isopropoxy. n is 0 or an integer of 1 to 5. When n is 2 or more, Z may be the same or different.

The compound of Formula (1) can be produced by reaction of a compound of Formula (2) with thioacetamide.

Examples of solvents used for the reaction include aromatic hydrocarbons such as benzene, toluene and xylene; ethers such as dioxane; nitriles such as acetonitrile; alcohols such as n-propyl alcohol, isopropyl alcohol and ethyl alcohol; and aliphatic carboxylic acids such as acetic acid and propionic acid. It is preferable to use an acid catalyst when a solvent other than a solvent of aliphatic carboxylic acid type is used.

Examples of suitable acid catalysts for use include sulfonic acids such as p-toluenesulfonic acid, and aliphatic carboxylic acids such as acetic acid. An acid catalyst is not required if an aliphatic carboxylic acid, such as acetic acid, is used as a solvent.

A reaction temperature suitable for the reaction system can be arbitrarily chosen. It usually ranges from room temperature to the boiling point of a solvent used. The reaction is usually completed in 1 hour to 24 hours.

The compound of Formula (1) may have tautomers shown in the following equation. These isomers are all covered by the present invention.

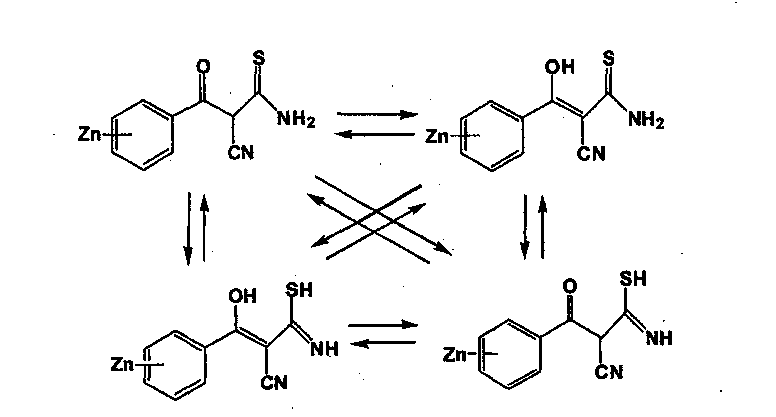

Actual examples of the compounds of the present invention are shown in Table 1.

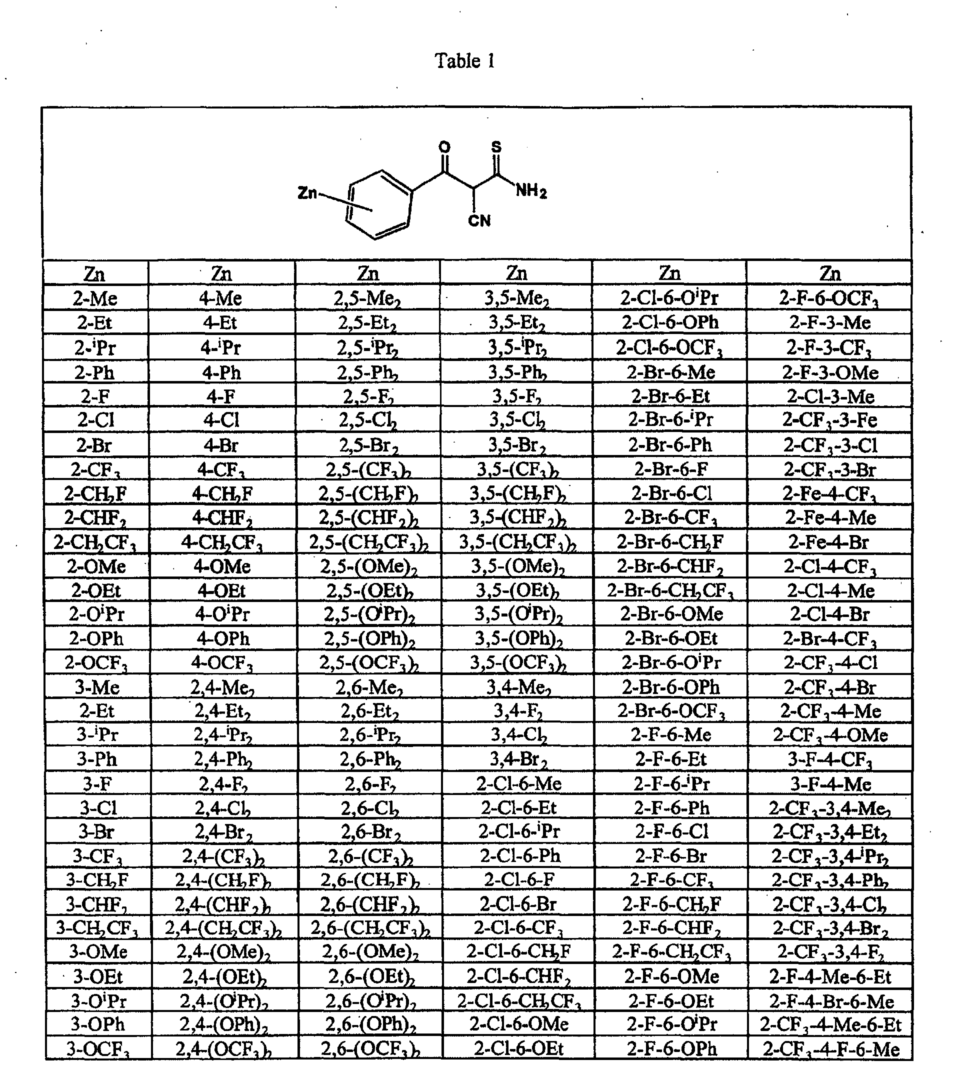

In the following are described processes for the preparation of benzoyl-substituted cyanomethylthiazoles (4) by that benzoyl-substituted cyanothioacetamide compounds of Formula (1) of the present invention that are produced according to the method described above are reacted with phenacyl halides of Formula (3).

In Formula (3), Y is halogen such as fluorine, chlorine, bromine or iodine; C₁₋₆ alkyl such as methyl, ethyl, n-propyl, isopropyl, n-butyl, sec-butyl, isobutyl, t-butyl, n-pentyl or its isomer, or n-hexyl or its isomer; C₁₋₆ alkoxy such as methoxy, ethoxy, n-propoxy, isopropoxy, n-butoxy, sec-butoxy, isobutoxy or t-butoxy; or optionally substituted phenyl. m is 0 or an integer of 1 to 5. When m is 2 or larger, Y may be the same or different. Examples of substituents on the phenyl or phenoxy group include halogen such as fluorine, chlorine, bromine and iodine; C₁₋₆ alkyl such as methyl, ethyl, n-propyl, isopropyl, n-butyl and t-butyl; C₁₋₆ haloalkyl such as chloromethyl, trifluoromethyl, trifluoroethyl and pentafluoroethyl; and C₁₋₆ alkoxy such as methoxy and ethoxy. The said phenyl or phenoxy group may have two or more, the same or different, substituents at arbitrary positions of the benzene ring.

X is halogen such as chlorine, bromine or iodine.

The reaction is carried out by stirring an organic solvent solution (or suspension) of compounds of Formula (1) and (3) at a specified temperature for a fixed period of time.

An amount of a compound of Formula (3) used usually ranges from 0.5 to 5 mole equivalents, preferably 1 to 3 mole equivalents, to a mole of a compound of Formula (1).

Examples of suitable solvents for use include alcohols such as methyl alcohol, ethyl alcohol, n-propyl alcohol, isopropyl alcohol, n-butyl alcohol, isobutyl alcohol and t-butyl alcohol; ethers such as diethyl ether, tetrahydrofuran, dioxane and 1,2-dimethoxyethane; nitrites such as acetonitrile and benzonitrile; and aromatic hydrocarbons such as benzene, toluene and xylene. These can be used alone or as a mixed solvent of two or more.

A reaction temperature is usually in a range of room temperature to the boiling point of a solvent used.

In the present invention, hydrogen halide is generated in the reaction system while the reaction proceeds. A base that captures the generated hydrogen halide is not always required to be present in the reaction system. However, if necessary, an organic base such as triethylamine or an inorganic base such as sodium hydrogen carbonate can be used to remove (neutralize) hydrogen halide.

Upon the completion of the reaction, ordinary work-up gives the target compound of Formula (4).

The compound of Formula (4) may have, for example, tautomers shown in the following equation. These are all covered by the present invention.

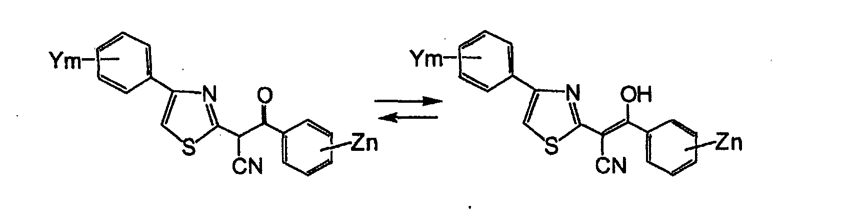

### Best Form to Implement the Invention:

The present invention is described in detail in reference to Examples, but not limited to the following process examples.

### Example 1: Preparation of á-(2-trifluoromethylbenzoyl)-á-cyanothioacetamide

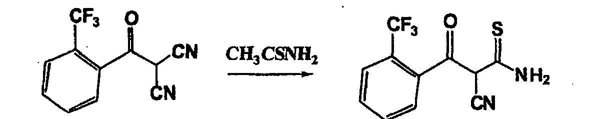

5g of 2-trifluoromethylbenzoylmalononitrile, 1.73g of thioacetamide and 0.5g of p-toluenesulfonic acid hydrate were dissolved in 50 ml of toluene. The resulting solution was refluxed for 1.5 hours. The reaction mixture was cooled to room temperature and poured into 100 ml of water. The toluene layer was separated. A 1N aqueous alkali was added to the toluene layer to stir well. The aqueous layer was separated and adjusted to pH = 1 with hydrochloric acid. The deposited crystals were separated by filtration and dried to give 4.85g of the target compound as crystals. Yield: 84.9%. Melting point: 103 to 105 C

### Example 2: Preparation of á-(2-trifluoromethylbenzoy)-á-cyanothioacetamide

3g of 2-trifluoromethylbenzoylmalononitrile and 0.95g of thioacetamide were dissolved in 10 ml of acetic acid. The resulting solution was refluxed for an hour. Acetic acid was distilled out from the reaction solution under reduced pressure. The obtained residue was poured into 50 ml of water and diethyl ether was added. The ether layer was separated, washed with brine, dried over anhydrous magnesium sulfate, and filtered. The filtrate was concentrated under reduced pressure. The obtained crude crystals were purified to give 0.4g of the target compound. Yield: 70.0%.

### Example 3: Preparation of á-(2-trifluoromethylbenzoyl)-á-cyanothioacetamide

0.3g of 2-trifluoromethylbenzoylmalononitrile, 0.1g of thioacetamide and 0.1g of p-toluenesulfonic acid monohydrate were dissolved in 3 ml of dioxane. The resulting solution was refluxed for 1.5 hours. The reaction mixture was cooled to room temperature and poured into aqueous alkali, and chloroform was added. The aqueous layer was separated and adjusted to pH = 1. The deposited crystals were separated by filtration and dried to give 0.28g of the target compound as crystals. Yield: 81.6%.

### Example 4: Preparation of á-(4-chlorobenzoyl)-á-cyanothioacetamide

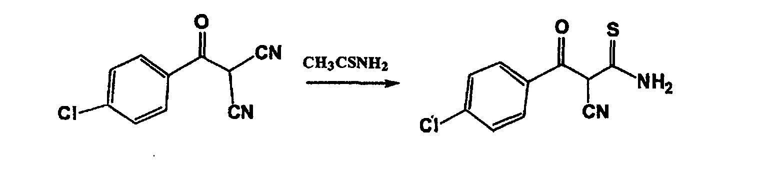

The preparation of this compound was carried out in the same method as described in Example 1 except that 4-chlorobenzoylmalononitrile was used. Yield: 85%. Melting point: 208 to 210 C (decomposed).

### Example 5: Preparation of á-(4-methylbenzoyl)-á-cyanothioacetamide

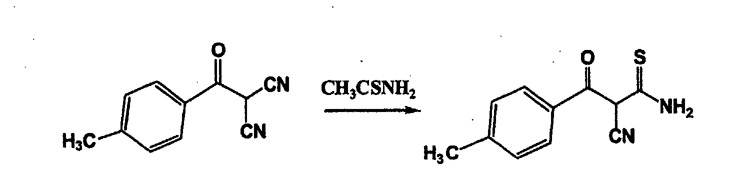

The preparation of this compound was carried out in the same method as described in Example 1 except that 4-methylbenzoylmalononitrile was used. Yield: 61%. Melting point: 181 to 183 C.

### Comparative Example 1: Preparation of á-(2-trifluoromethylbenzoyl)-á-cyanothioacetamide

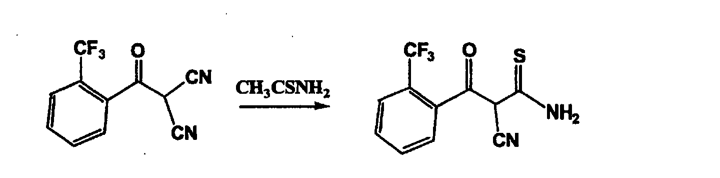

Hydrogen chloride gas was blown into a solution of 3g of 2-trifluoromethylbenzoylmalononitrile and 0.95g of thioacetamide in 10 ml of DMF at 100 C for 30 minutes. The reaction solution was poured into 50 ml of ice-water, and extracted with 100 ml of diethyl ether. The ether layer was washed with brine, dried over anhydrous magnesium sulfate and filtered. The filtrate was concentrated under reduced pressure. The obtained residue was dissolved in a solvent of n-hexane and ethyl acetate (4:1), and purified through a silica gel short column to give 1.5g of the target compound. Yield: 43.8%.

### Comparative Example 2

The same method describe d in Example 1 was carried out by using DMF instead of toluene. The solution was heated at 100 C with stirring for 6 hours to give the target compound in yield of about 20 to 30%.

### Example 6: Preparation of 2-[4-(2,6-difluorophenyl)-(1,3-thiazol-2-yl)]-3-oxo-3-[2-(trifluoromethyl)phenyl]propanenitrile

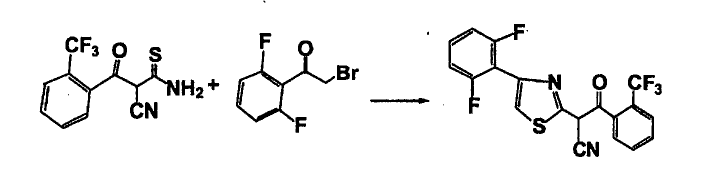

0.6g (2.2 mmoles) of á-(2-trifluoromethylbenzoyl)cyanothioacetamide was dissolved in 5 ml of ethyl alcohol, and 0.5g (2.1 mmoles) of á-bromo-2,6-difluoroacetophenone was dropped at room temperature. The resulting solution was stirred at room temperature for 2 hours and at 50 C for 7 hours. The reaction solution was cooled to room temperature, poured into aqueous hydrochloric acid, and extracted with ethyl acetate. The ethylacetate layer was washed with water and with brine in this order, dehydrated over anhydrous magnesium sulfate and concentrated under reduced pressure. The obtained residue was purified through silica gel chromatography to give 0.55g of the target compound. Yield: 63.2%. Melting point: 186 to 188 C

### Example 7: Preparation of 2-[4-(2,6-difluorophenyl)-(1,3-thiazol-2-yl)]-3-oxo-3-[2-(trifluoromethyl)phenyl]propanenitrile

0.6g (2.2 mmoles) of á-(2-trifluoromethylbenzoyl)cyanothioacetamide was dissolved in 5 ml of ethyl alcohol, and 0.23g (2.2 mmoles) of triethylamine was added at room temperature The resulting solution was stirred for 15 minutes. Into the resulting solution was dropped 0.5g (2.1 mmoles) of á-bromo-2,6-difluoroacetophenone at room temperature (exothermic), and stirred for 2 hours. The reaction solution was poured into cold aqueous hydrochloric acid, and extracted with ethyl acetate. The obtained product was purified in the same way as that used in Example 6 to give 0.81g of the target compound. Yield: 93.3%.

### Applicability in Industry:

As described above, the á-benzoyl-substituted-á-cyanothioacetamides of the present invention are useful as intermediates for producing agricultural chemicals and medicine, particularly valuable as intermediates for the preparation of the insecticides disclosed in WO 00/17174.

The production processes of the present invention produce, advantageously at an industrial scale, á-benzoyl-substituted-á-cyanothioacetamides and also 3-oxo-2-(2-thiazolyl)-3-phenylpropanenitrile compounds that are intermediates for producing the insecticides disclosed in WO 00/17174.

## Claims

1. A compound represented by Formula (1) 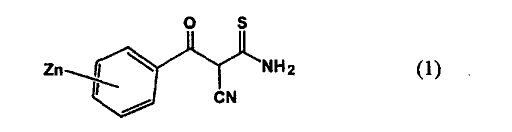 (wherein, Z is nitro, cyano, halogen, alkyl having 1 to 6 carbons, haloalkyl having 1 to 6 carbons, alkoxy having 1 to 6 carbons, optionally substituted phenyl or optionally substituted phenoxy; and n is 0 or an integer of 1 to 5).

2. A process for producing the benzoyl-substituted cyanothioacetamide derivatives of Formula (1) 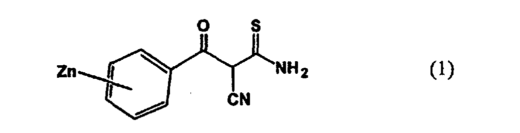 (wherein, Z and n are as defined above) **characterized in that** a malononitrile whose benzoyl group is substituted and represented by Formula (2) 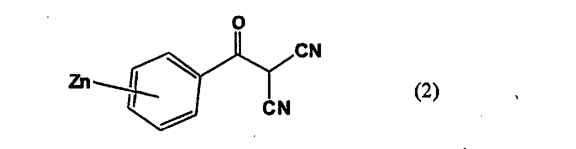 (wherein, Z and n are as defined in Claim 1) is reacted with thioacetamide represented by Formula CH₃CSNH₂.

3. A process for the preparation of a compound represented by Formula (4) 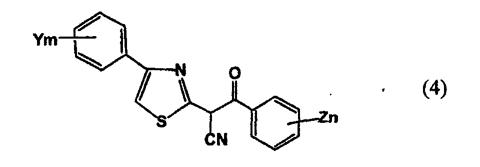 (wherein, Y is halogen, alkyl having 1 to 6 carbons, alkoxy having 1 to 6 carbons or optionally substituted phenyl; m is 0 or an integer of 1 to 5; and Z and n are as defined above) **characterized in that** a compound of Formula (1) 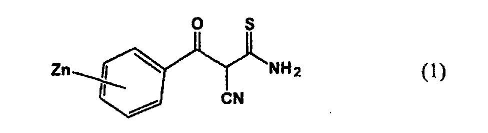 (wherein, Z and n are as defined above) is reacted with a phenacyl halide represented by Formula (3) 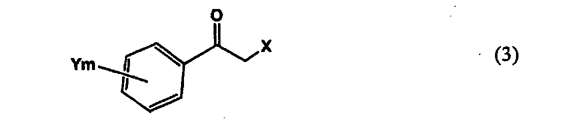 (wherein, X is halogen, and Y and m are as defined above).
